# FASCICULE DE BREVET EUROPEEN

(11) **EP 0 751 743 B1**
(45) Date de publication et mention de la délivrance du brevet: **26.05.1999**
(21) Numéro de dépôt: 95914421.3
(22) Date de dépôt: 24.03.1995
(51) Int. Cl.: A61B 8/08

(54) **PROCEDE ET DISPOSITIF D'EVALUATION ET DE CARACTERISATION DES PROPRIETES DES OS**
VERFAHREN UND GERÄT ZUR AUSWERTUNG UND CHARAKTERISIERUNG VON KNOCHENEIGENSCHAFTEN
METHOD AND DEVICE FOR ESTIMATING AND CHARACTERISING BONE PROPERTIES

(30) Priorité: 25.03.1994 FR 9403555
(43) Date de publication de la demande: 08.01.1997
(73) Titulaire: CENTRE NATIONAL DE LA RECHERCHE SCIENTIFIQUE (CNRS), 75794 Paris Cédex 16 (FR)
(72) Inventeur: LAUGIER, Pascal, F-75017 Paris (FR); BERGER, Geneviève, F-92340 Bourg-la-Reine (FR)
(74) Mandataire: Armengaud Ainé, Alain
(86) Numéro de dépôt international: FR9500376
(87) Numéro de publication internationale: WO9526160

(56) Documents cités:
- WO-A-90/01296
- PHYSICS IN MEDICINE & BIOLOGY, vol. 35, no. 10, Octobre 1990 BRISTOL, GB, pages 1387-1396, J.A.EVANS ET AL 'ultrasonic attenuation and velocity in bone'
- PROCEEDINGS OF IEEE 1990 ULTRASONICS SYMPOSIUM, vol. 3, 4 Décembre 1990 HONOLULU, US, pages 1367-1370, N.CHUBACHI ET AL 'measurement of bone properties by ultrasound to develop diagnostic equipment'

## Description

La présente invention est relative à un procédé d'évaluation et de caractérisation in vivo des propriétés mécaniques ou architecturales des os, mettant en oeuvre la technique des ultrasons, en vue de l'obtention d'images paramétriques.

Au cours des dernières années sont apparus sur le marché des appareils ultrasonores permettant la mesure de l'atténuation et de sa dépendance en fonction de la fréquence (coefficient d'atténuation), ou de la vitesse de propagation des ultrasons en transmission à-travers le calcanéum ou la rotule. Ces dispositifs sont essentiellement destinés au dépistage ou au suivi de l'ostéoporose.

On connaît déjà un certain nombre de dispositifs assurant l'évaluation de l'os in vivo par la mise en oeuvre de faisceaux d'ultrasons.

Les publications WO 90/01903 et WO 87/07494 décrivent des appareils permettant d'effectuer une mesure de la vitesse de propagation des ultrasons dans l'os en utilisant deux transducteurs qui sont placés de part et d'autre de l'os.

US-A-4 774 959 décrit un dispositif assurant la mesure du coefficient d'atténuation en transmission à l'aide d'une première paire de transducteurs, ce dispositif comportant une seconde paire de transducteurs assurant une mesure de l'épaisseur de l'os à l'endroit où le faisceau ultrasonore a traversé l'os.

EP-A-0341 969 et O 480 554 décrivent des dispositifs permettant la mesure du coefficient d'atténuation en transmission et de la vitesse des ultrasons à l'aide de transducteurs placés face à face.

Enfin US-A-4 941 474 décrit un appareil permettant d'analyser à la fois des signaux transmis à travers l'os et des signaux qui sont réfléchis ou diffusés par l'architecture interne de l'os. Cet appareil comporte au moins un transducteur émettant un faisceau ultrasonore au-travers de l'os, des moyens pour recevoir et mettre en mémoire les signaux transmis au-travers de l'os ou réfléchis par les faces de celui-ci et des moyens pour traiter les signaux ainsi stockés en vue de la mesure de la vitesse de propagation du faisceau ultrasonore dans l'os, de l'épaisseur de ce dernier, du coefficient d'atténuation en transmission du faisceau ultrasonore et des paramètres de réflexion. Cet appareil ne permet pas de réaliser des images paramétriques.

L'expérience découlant de l'utilisation des appareils connus mentionnés ci-dessus démontre que les mesures qu'ils permettent d'effectuer restent rudimentaires et que la précision, la sensibilité et la reproductibilité de ces mesures doivent être améliorées. En outre, aucun de ces appareils connus ne permet l'obtention d'images paramétriques.

Partant de cet état de la technique, l'invention apporte un procédé du type défini dans US-4 941 474, mais permettant d'obtenir des images paramétriques.

Selon ce procédé :
- on utilise au moins un transducteur focalisé émettant un faisceau ultrasonore au-travers de l'os ;
- on réalise un balayage dudit faisceau dans un plan perpendiculaire à la direction de propagation du faisceau ultrasonore ;
- on recueille les signaux transmis au-travers de l'os et/ou réfléchis par les faces de l'os et/ou diffusés par les structures internes de l'os ;
- on met en mémoire les signaux obtenus et,
- on traite les signaux ainsi stockés pour la mesure de la vitesse de propagation du faisceau ultrasonore dans l'os, de l'épaisseur de l'os, du coefficient d'atténuation en transmission du faisceau ultrasonore et/ou des paramètres de réflexion pour l'estimation des coefficients de rétro-diffusion et d'atténuation.

Selon la présente invention, pour la réalisation, en mode transmission, d'images paramétriques d'atténuation en fonction de la fréquence et de la vitesse de propagation des ultrasons on met en oeuvre une paire de transducteurs focalisés, placés en vis à vis et fonctionnant à basse fréquence, c'est-à-dire à une fréquence centrale comprise entre 100 kHz et 3 MHz, et de préférence entre 100 kHz et 1 MHz.

Dans son application à la réalisation en mode échographique (mode réflexion) d'images de réflectivité, le procédé objet de l'invention utilise un transducteur focalisé ou une paire de transducteurs focalisés, placés en vis à vis, et pouvant fonctionner dans une gamme de fréquences plus étendue, c'est-à-dire à une fréquence centrale comprise entre 100 kHz et 10 MHz.

Selon la présente invention, pour la réalisation en mode échographique d'images de réflectivité et pour l'estimation des coefficients d'atténuation et/ou de rétrodiffusion en fonction de la fréquence du faisceau ultrasonore, et également en vue de la réalisation, en transmission, d'images paramétriques d'atténuation en fonction de la fréquence et de la vitesse de propagation, on met en oeuvre une paire de transducteurs placés en vis à vis et fonctionnant à basse fréquence, c'est-à-dire à une fréquence centrale comprise entre 100 kHz et 3 MHz, de préférence entre 100 kHz et 1 MHz et un transducteur focalisé ou une paire de transducteurs focalisés, placés en vis à vis et fonctionnant à haute fréquence, c est-à-dire à une fréquence centrale comprise entre 1 MHz et 10 MHz.

L'invention vise en second lieu un dispositif tel que defini dans la revendication 7.

Selon un exemple de réalisation du dispositif objet de l'invention, on utilise des transducteurs focalisés, placés en vis-a-vis, ces transducteurs étant du type monoélément.

Selon un autre mode de réalisation de la présente invention, les mesures peuvent être effectuées en immersion, le dispositif comportant alors une enceinte remplie d'un liquide tel que de l'eau, ou bien ces mesures peuvent être effectuées par contact à l'aide d'un milieu de couplage et en utilisant un réseau de transducteurs ultrasonores.

Ce dispositif est en outre caractérisé en ce que le module de traitement de signal comporte un ordinateur, une mémoire de masse et un ensemble de logiciels de traitement de signaux conçus pour l'exploitation des données provenant du module d'acquisition, ce module de traitement comportant un ensemble de fonctions permettant notamment d'effectuer le calcul du coefficient d'atténuation en transmission, le calcul de l'épaisseur de l'os traversé à l'endroit de la mesure, le calcul de la vitesse de propagation, le calcul du coefficient de rétrodiffusion en réflexion et le calcul du coefficient d'atténuation en réflexion.

D'autres caractéristiques et avantages de la présente invention ressortiront de la description faite ci-après en référence aux dessins annexés qui en illustrent divers exemples de mise en oeuvre et de réalisation, dépourvus de tout caractère limitatif. Sur les dessins ;
- la figure 1 est une vue schématique en élévation représentant un exemple de réalisation d'un appareil pour la mise en oeuvre du procédé objet de l'invention ;
- la figure 2 est un schéma par blocs illustrant, d'une façon générale, le module d'acquisition des données mis en oeuvre par l'invention ;
- la figure 3 est également un schéma illustrant d'une façon générale un module d'acquisition fonctionnant à la fois en transmission et en réflexion ;
- la figure 4 est un schéma illustrant le principe de l'acquisition en transmission ;
- la figure 5 est un schéma qui illustre le principe général de l'acquisition en transmission et en réflexion ;
- la figure 6 est un schéma illustrant une variante du mode d'acquisition en transmission et réflexion ;
- la figure 7 est un schéma par blocs illustrant le principe général du traitement des signaux dans le procédé et le dispositif objet de l'invention ;
- la figure 8 est un schéma illustrant le traitement du signal transmis en vue de la mesure de la vitesse de propagation de l'onde ultrasonore dans l'os ;
- la figure 9 est encore un schéma illustrant le traitement du signal, en mode échographique, en vue de la mesure de l'épaisseur de l'os et,
- la figure 10 est un schéma par blocs illustrant le principe du traitement du signal transmis pour la mesure de l'atténuation.

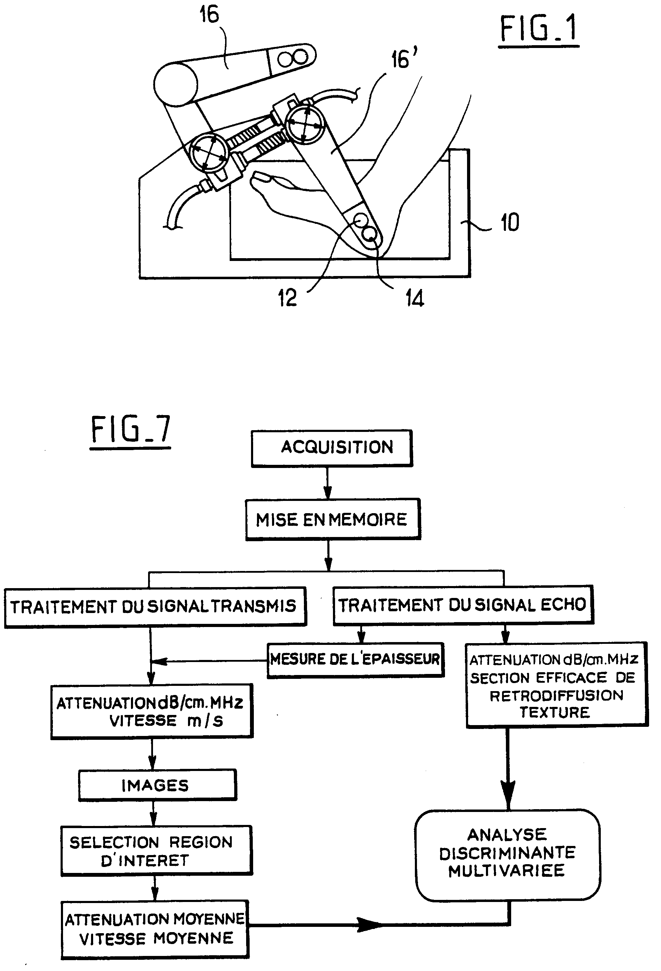

En se référant à la figure 1, on voit que, dans cet exemple de réalisation non limitatif, le dispositif mettant en oeuvre le procédé objet de l'invention comporte essentiellement une enceinte 10 remplie d'eau permettant de réaliser les mesures en immersion, la présence du milieu liquide assurant un bon couplage entre la source de rayonnement et l'os dont on veut évaluer notamment les propriétés mécaniques. Ce dispositif comporte une source d'émission d'ultrasons, constituée ici de paires de transducteurs ultrasonores piézoélectriques focalisés, placés en vis à vis de part et d'autre de l'os à analyser, l'une des paires de transducteurs tels que 12 fonctionnant à haute fréquence, c'est-à-dire dans le domaine de fréquences de 1 MHz à 10 MHz, spécifié ci-dessus, alors que l'autre paire de transducteurs tels que 14, fonctionnent à basse fréquence dans un domaine compris entre 100 kHz et 1 MHz. Ces paires de transducteurs sont montées sur un pont mobile qui sur la figure 1 a été représenté respectivement en position relevée 16 et en position abaissée 16' pour la mesure.

Ainsi qu'on l'a spécifié ci-dessus, le principe sur lequel repose le procédé objet de l'invention réside dans la transmission d'une onde ultrasonore dans l'os et dans l'étude de l'interaction de cette onde avec l'os. Le dispositif selon l'invention illustré par la figure 1 peut fonctionner à la fois en transmission et en réflexion. Dans cet exemple de réalisation, il est muni d'un système classique de balayage du faisceau ultrasonore, ce balayage étant obtenu par déplacement des transducteurs focalisés sous l'action de deux moteurs assurant les déplacements dans un plan, selon les axes orthogonaux X et Y, permettant ainsi une exploration de la totalité du volume osseux.

Le dispositif comporte en outre un module d'acquisition chargé de l'émission des signaux ultrasonores et de la réception de ces signaux après leur interaction dans l'os. On prévoit également un système de mise en mémoire des signaux ainsi obtenus, et un module de traitement de ces signaux pour l'estimation de certains paramètres acoustiques connus pour leur relation avec les propriétés mécaniques ou visco-élastiques de l'os.

L'invention, grâce notamment à un balayage automatique du faisceau ultrasonore apporte une solution aux difficultés résultant de l'utilisation des appareils actuels, notamment en ce qui concerne un positionnement précis de l'os pour la mesure et la localisation de la région où s'effectue la mesure. L'invention permet en outre d'effectuer des mesures ultrasonores en transmission à travers l'os et en réflexion. Le recueil des signaux réfléchis et/ou diffusés par l'architecture interne de l'os apporte une information complémentaire à celle qui est déjà contenue dans les signaux qui ont été transmis à travers l'os. Ainsi le dispositif selon l'invention permet d'obtenir une quantité d'informations bien supérieure à celle obtenue par les dispositifs de types connus. En outre, l'invention apporte une bien meilleure précision.

Comme on l'a spécifié dans le préambule de la présente description, le procédé objet de l'invention permet d'obtenir, en mode transmission, des images paramétriques d'atténuation en fonction de la fréquence et de la vitesse de propagation du faisceau ultrasonore, en mettant en oeuvre une paire de transducteurs focalisés, placés en vis à vis et fonctionnant à basse fréquence, c'est-à-dire à une fréquence centrale comprise entre 100 kHz et 1 MHz.

L'invention permet également de réaliser, en mode échographique, des images de réflectivité et d'obtenir une estimation des coefficients d'atténuation et de rétrodiffusion, en utilisant un seul transducteur focalisé ou une paire de transducteurs focalisés, placés en vis à vis, et fonctionnant à haute fréquence, c'est-à-dire à une fréquence centrale comprise entre 1 MHz et 10 MHz.

Enfin, on peut également grâce au procédé de l'invention, réaliser des images de réflectivité en mode échographique et obtenir une estimation moyenne des coefficients d'atténuation et/ou de rétrodiffusion en fonction de la fréquence du faisceau ultrasonore, et également réaliser en transmission des images paramétriques d'atténuation en fonction de la fréquence et de la vitesse de propagation du faisceau ultrasonore. Dans cette application, l'invention met en oeuvre une paire de transducteurs placés en vis à vis et fonctionnant à basse fréquence, c'est-à-dire à une fréquence centrale comprise entre 100 kHz et 1 MHz, et une paire de transducteurs focalisés, placés en vis à vis et fonctionnant à haute fréquence, c est-à-dire à une fréquence centrale comprise entre 1 MHz et 10 MHz.

Ainsi qu'on l'a déjà spécifié ci-dessus le dispositif mettant en oeuvre le procédé selon l'invention comporte essentiellement un module 〈〈 Acquisition du signal ultrasonore 〉〉 et un module 〈〈 Traitement du signal 〉〉.

Le module 〈〈 Acquisition 〉〉 a pour fonctions :
- l'émission des ondes ultrasonores à l'aide des transducteurs définis ci-dessus ;
- la réception d'ondes ultrasonores, dites de référence, la réception d'ondes ultrasonores transmises à travers l'os, la réception d'ondes réfléchies et/ou diffusées par l'os et ses structures ;
- le balayage de tout le volume osseux par le faisceau ultrasonore.

Ce module 〈〈 Acquisition 〉〉 se compose comme défini ci-dessus des sources et récepteurs ultrasonores (paire de transducteur focalisés).L'impulsion ultrasonore ainsi rayonnée est transmise dans le milieu de propagation avec lequel elle interagit. Ainsi qu'on l'a spécifié ci-dessus, la mesure peut être faite en immersion pour obtenir un bon couplage entre la source de rayonnement et l'os. L'interaction entre le milieu de propagation et l'onde incidente donne naissance à une onde directement transmise (onde cohérente) et à une ou plusieurs ondes réfléchies ou diffusées par l'os et/ou ses structures internes.

Ce module 〈〈 Acquisition 〉〉 se compose en outre d'un étage de détection, amplification (par exemple contrôle automatique de gain) et de conversion analogique-digitale des signaux, d'un ordinateur, et d'un processeur avec son environnement classique. Cette partie du dispositif est de conception classique et en conséquence elle ne sera pas décrite.

Ce module comporte en outre, dans cet exemple de réalisation, le dispositif mécanique assurant le balayage du faisceau ultrasonore dans un plan perpendiculaire à la direction de propagation des faisceaux ultrasonores. Lors du balayage, l'émetteur et le récepteur ultrasonores effectuent le même mouvement, de façon synchrone, si bien que leur position relative reste identique. Ce balayage est assuré à l'aide de deux moteurs programmables.

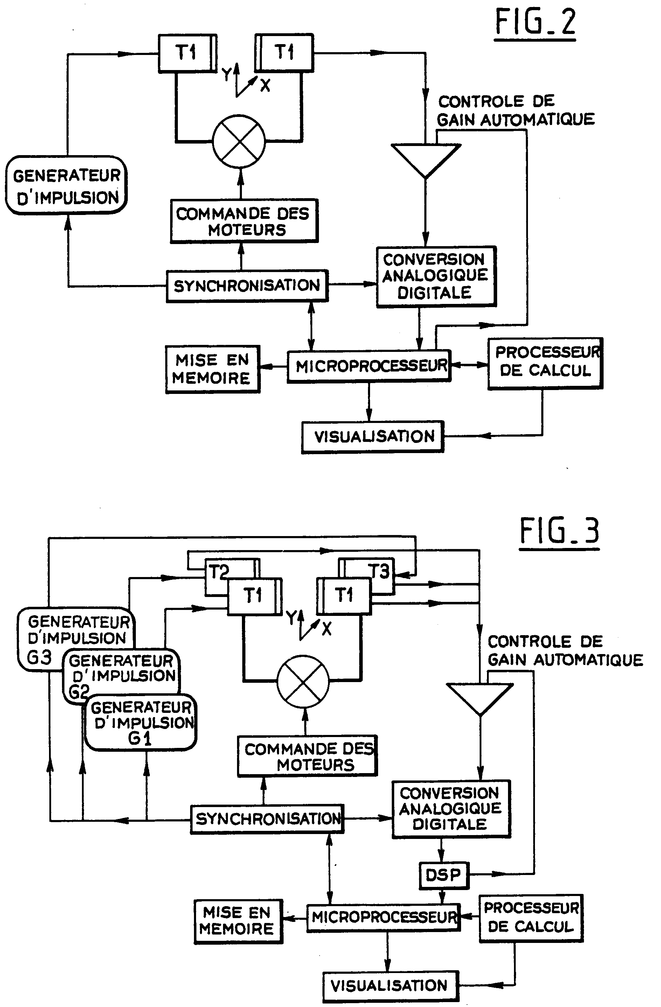

La figure 2 est un schéma général du dispositif selon l'invention assurant un fonctionnement uniquement en mode transmission. Ce dispositif comporte un transducteur T1 pour l'émission, associé à un transducteur T'1 pour la réception, disposé en vis-à-vis. Les transducteurs sont focalisés, la distance qui les sépare étant égale environ au double de la distance focale. Le transducteur T'1 détecte les ondes émises par le transducteur T1, et les transforme en signal électrique qui est amplifié, numérisé, et transféré sur l'ordinateur.

La figure 3 représente un schéma général d'un dispositif fonctionnant à la fois en mode transmission et réflexion. Un transducteur ultrasonore T2 ou T3 est excité périodiquement de manière à rayonner une onde ultrasonore dans le milieu de propagation. Cette onde interéagit avec le milieu de propagation et elle subit une réflexion partielle et/ou une diffusion. Une partie de l'énergie incidente est réfléchie à l'entrée de l'os, une partie de l'énergie transmise dans l'os est ensuite diffusée par les structures internes de ce dernier et notamment rétrodiffusée vers le transducteurémetteur T2 ou T3. C'est ce même transducteur T2 ou T3 qui sert de récepteur pour détecter les ondes réfléchies par l'os et rétrodiffusées par l'architecture interne de ce dernier et pour les transformer en signal électrique qui est ensuite amplifié, numérisé, et transféré sur l'ordinateur. Le transducteur T2 ou T3 est relié électriquement à l'étage de réception. L'utilisation combinée en réflexion de deux transducteurs T2 et T3, placés en vis à vis, permet de détecter simultanément les échos de deux faces opposées de l'os. On peut ainsi effectuer une mesure automatique ultrasonore de l'épaisseur de l'os. De plus, un traitement de signal peut être effectué ultérieurement sur les signaux rétrodiffusés et enregistrés par les transducteurs T2 ou T3 afin d'en extraire des paramètres acoustiques utiles pour caractériser l'os : atténuation en fonction de la fréquence, section efficace de rétrodiffusion, paramètre de texture etc...

Pour fonctionner simultanément en transmission et en réflexion avec deux paires de transducteurs placées en vis à vis, on utilise de préférence la solution illustrée par la figure 3 selon laquelle on met en oeuvre trois étages de générateurs d'impulsions pour commander les transducteurs T1, T2 et T3, les excitations étant séquentielles dans le temps : T1 émet le premier, lorsque T'1 a détecté l'onde émise par T1, T2 émet et il détecte les signaux réfléchis et rétrodiffusés. Enfin T3 est excité à son tour.

Une autre solution plus économique mais moins rapide consiste à n'utiliser qu'un seul générateur d'impulsions commun pour l'excitation des transducteurs T1, T2 et T3. Dans ce cas, il suffit de prévoir un commutateur pour diriger l'impulsion excitatrice vers un transducteur différent à chaque tir. Ce commutateur est relié au microprocesseur qui contrôle la commutation. Cette variante n'a pas été représentée.

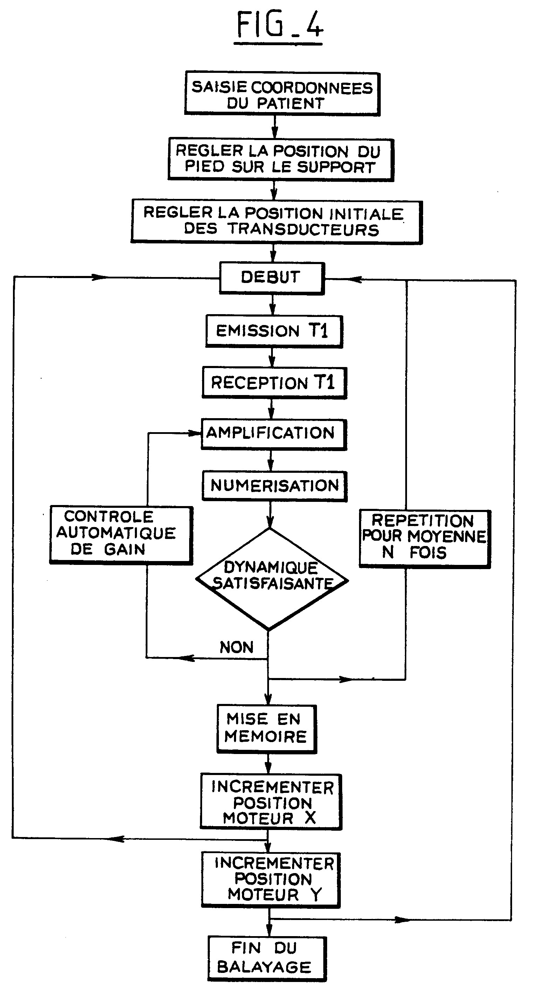

Sur la figure 4, on a représenté le diagramme de l'acquisition par transmission. Les différentes étapes de cette acquisition ressortent clairement de l'examen de cette figure.

On peut utiliser deux solutions pour l'acquisition des données en transmission et en réflexion.

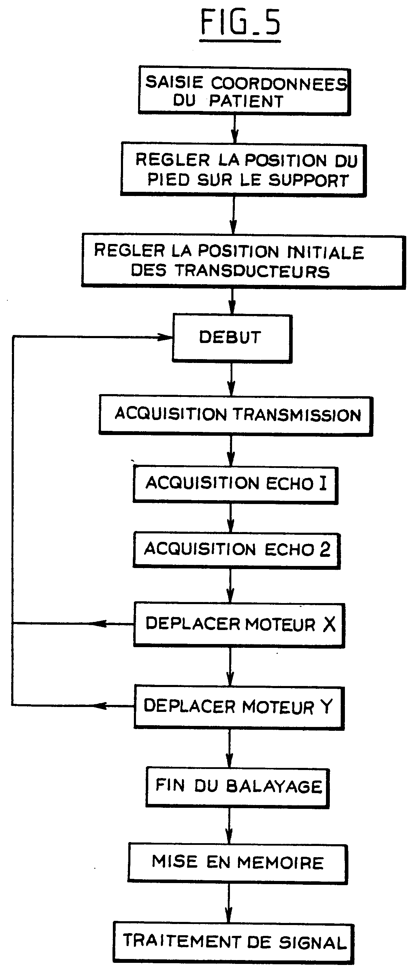

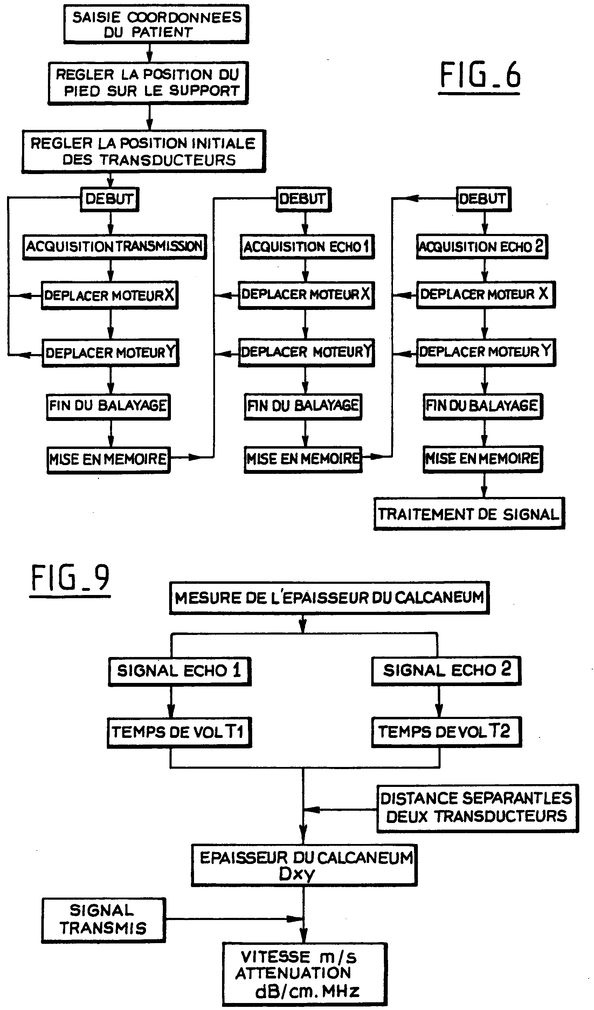

Dans la première solution illustrée par la figure 5 les données transmises et réfléchies sont enregistrées au cours d'un balayage unique. Pour chaque position X,Y, des transducteurs T1, T2, T3 sont excités successivement. Dans la seconde solution illustrée par la figure 6, les données transmises et réfléchies sont enregistrées au cours de trois balayages différents : le premier balayage est destiné à l'acquisition des données par la paire des transducteurs T1 et T'1 et les deuxième et troisième balayages sont destinés à l'acquisition des données en réflexion par les transducteurs T2 et T3.

Il est possible d'acquérir plusieurs signaux à la suite pour une position fixée des transducteurs. L'amélioration du rapport signal/bruit est obtenue par l'intermédiaire de la moyenne de ces différentes acquisitions. Dans le cas où cette possibilité existe, l'émission-réception est répétée autant de fois que cela est nécessaire. Une fois cette séquence d'acquisition terminée, la position des moteurs est incrémentée et l'opération peut être répétée.

Dès que la phase d'acquisition des signaux est terminée, tous les signaux numérisés sont mis en mémoire et la phase de traitement de signal peut alors commencer, soit de façon automatique. soit par l'intermédiaire d'un ordre de commande lancé par l'opérateur.

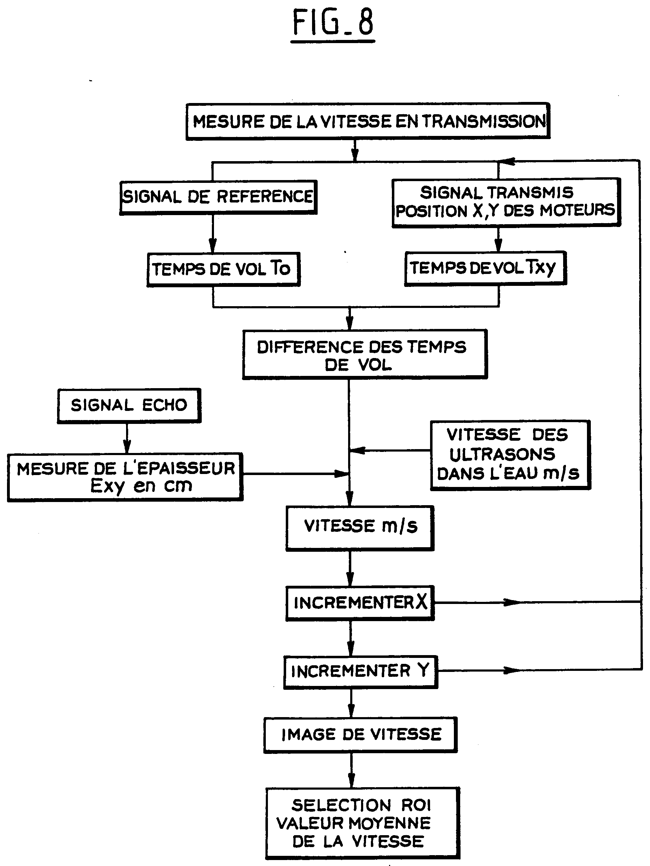

Le dispositif mettant en oeuvre la présente invention comporte, ainsi qu'on l'a précisé ci-dessus, un module de traitement de signal. Le principe général du traitement des signaux est illustré par le schéma de la figure 7.

Ce module de traitement de signal comporte un ordinateur, une mémoire de masse et un ensemble de logiciels de traitement de signaux conçus pour l'exploitation des données précédemment acquises en transmission et/ou en réflexion. Il comporte un ensemble de fonctions préprogrammées permettant notamment d'effectuer les calculs suivants :
- calcul de l'atténuation (en dB) en fonction de la fréquence en transmission
- calcul du coefficient d'atténuation en fonction (en dB/MHz) de la fréquence en transmission
- calcul des temps de vol des signaux transmis à travers l'os
- calcul de l'épaisseur de l'os traversé à l'endroit de la mesure
- calcul du coefficient d'atténuation en fonction de la fréquence (dB/cm.MHz) en transmission
- calcul de la vitesse de propagation des ultrasons en transmission
- calcul de l'atténuation (en dB) en fonction de la fréquence en réflexion
- calcul du coefficient d'atténuation en fonction de la fréquence (dB/cm.MHz) en réflexion
- calcul de la section efficace de rétrodiffusion (en dB) en fonction de la fréquence en réflexion
- calcul du coefficient de rétrodiffusion (dB/MHz) en réflexion
- calcul du coefficient intégral de rétrodiffusion (dB.MHz) en réflexion.

Il est bien entendu possible d'ajouter d'autres fonctions de traitement de signal ou de traitement d'image susceptibles d'apporter une information quantitative utile à la caractérisation de l'os (analyse de texture par exemple).

Les paramètres sont obtenus pour chaque position des transducteurs, ce qui permet d'obtenir une cartographie des paramétres. Un traitement d'images est compris dans le logiciel permettant ainsi de sélectionner une ou plusieurs régions de la mesure, de forme quelconque, pour une estimation d'une moyenne locale des paramètres.

La méthode d'estimation des paramètres (atténuation et vitesse de propagation) en transmission repose sur la comparaison d'un signal de référence avec un signal transmis à travers l'os.

Le signal de référence est un signal qui s'est propagé dans un milieu dont les caractéristiques acoustiques (atténuation et vitesse) sont bien connues (l'eau dans cet exemple de réalisation). Le signal de référence et les signaux transmis dans l'os sont enregistrés dans les mêmes conditions. Par exemple, le signal de référence peut être acquis soit lors de la mise en route de l'appareil, soit avant chaque examen. On peut également enregistrer une référence unique et la stocker dans la mémoire de l'ordinateur pour la rappeler ensuite à chaque examen.

On décrira maintenant à titre d'exemple divers modes de traitement de signaux pour certaines fonctions réalisées par le procédé de l'invention.

### 1/ Estimation de la vitesse de propagation en transmission.

Le principe de traitement du signal transmis pour la mesure de cette vitesse est illustré par le schéma de la figure 8. Ce mode de traitement comporte donc les étapes suivantes :
- Les transducteurs sont à la position X, Y:
   Le transducteur T1 émet et l'onde ainsi émise, transmise à travers l'os, est détectée par le transducteur-récepteur T'1, puis amplifiée, numérisée, et transférée sur ordinateur. Bien entendu, et comme spécifié ci-dessus, pour des mesures à travers le calcanéum, les transducteurs et l'os sont placés dans un bain dont la température est contrôlée-par thermostat (voir la figure 1). Cette première mesure est destinée à l'estimation du temps de vol de l'onde transmise à travers l'os et au calcul de la différence entre ce temps de vol et celui du signal de référence;
- Le transducteur T2 (ou T1) émet et le transducteur T2 (ou T1) reçoit le signal ainsi émis et réfléchi par l'os. L'onde ultrasonore réfléchie est amplifiée, numérisée, et transférée sur ordinateur.
- On estime la durée du temps de vol de l'écho qui est réfléchi par la face d'entrée de l'os tournée vers le transducteur T2 (ou T1) :
- Le transducteur T3 (ou T'1) émet et le transducteur T3 (ou T'1) assure la réception du signal réfléchi par l'os. L'onde réfléchie est amplifiée, numérisée, et transférée sur ordinateur.
- On estime la durée du temps de vol de l'écho réfléchi par la face d'entrée de l'os, qui est tournée vers T3 (ou T'1).
- Ces deux dernières mesures sont destinées au calcul de l'épaisseur du calcaneum à l'endroit de la mesure. La vitesse de propagation en transmission de l'onde ultrasonore se déduit de manière connue du temps de vol de l'onde entre les deux transducteurs. Pour estimer la vitesse de propagation, il faut connaître l'épaisseur de l'os et celle-ci, selon l'invention, est mesurée par ultrasons.

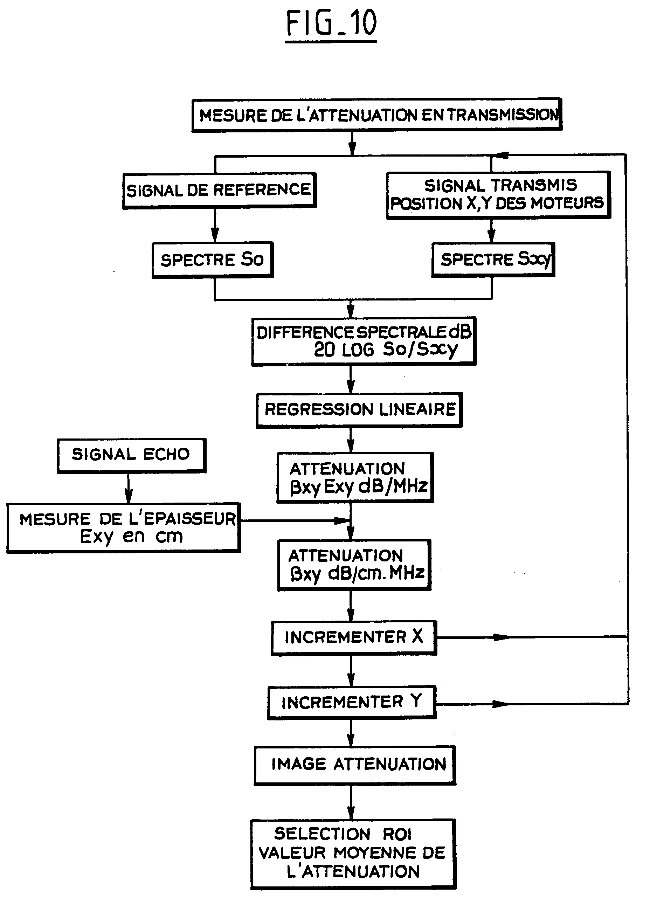

La figure 9 illustre le principe de traitement du signal échographique pour la mesure de cette épaisseur. En effet, par échographie on peut déterminer la distance séparant un transducteur de la face de l'os (face d'intérêt). Il suffit d'identifier l'écho de cette face et de mesurer son temps de vol. Chaque transducteur T2 et T3 (ou T1 et T'1) est interrogé à tour de rôle en mode échographique, et l'on mesure les temps de vol t1 et t2 des échos réfléchis par chacune des faces latérales opposées de l'os. On en déduit l'épaisseur de l'os.

### 2/ Estimation du coefficient d'atténuation en transmission.

Le principe du traitement de signal selon ce mode particulier est illustré par la figure 10. Il comporte les étapes suivantes :
- Les transducteurs sont initialement dans la position X,Y
- Le transducteur T1 émet et l'onde transmise à travers l'os est détectée par le transducteur-récepteur T'1, puis elle est amplifiée, numérisée et transférée sur ordinateur. Comme précédemment, pour des mesures à travers le calcaneum, dans cet exemple de réalisation, les transducteurs et l'os sont placés dans un bain dont la température est contrôlée par thermostat (fig. 1);
- On calcule les spectres en fréquence du signal transmis à travers l'os et du signal de référence. L'atténuation en fonction de la fréquence est obtenue par comparaison du spectre d'un signal de référence enregistré dans l'eau et de celui du signal transmis à travers l'os. Le signal ultrasonore est du type impulsionnel et il comporte plusieurs fréquences dans l'intervalle compris entre 0,2 MHz et 1 MHz.

Afin de s'affranchir des variations liées à l'épaisseur du calcaneum, il faut comparer les atténuations rapportées à l'épaisseur (dB/MHz.cm) exacte du calcaneum au point de mesure. On a exposé ci-dessus la manière selon laquelle on peut effectuer en mode échographique une mesure ultrasonore de l'épaisseur.

Comme pour tous les milieux hétérogénes, la valeur des paramètres acoustiques dépend de l'endroit où la mesure est effectuée. Le dispositif de balayage de l'appareil selon l'invention permet d'explorer tout le volume osseux et d'obtenir une cartographie des paramètres. A partir des images d'atténuation en fonction de la fréquence et de la vitesse de l'onde ultrasonore. on peut sélectionner une région de la mesure (région d'intérêt) et estimer une moyenne des paramètres dans cette région. Le logiciel comprend quelques fonctions simples de traitement d'images, notamment pour la sélection des régions de mesure, de forme et de taille variables. Le support de l'image est utilisé pour sélectionner des régions de mesures identiques chez des patients différents ou lors de mesures répétées chez un même patient.

Ainsi, l'invention permet d'obtenir notamment une imagerie ultrasonore du calcaneum (images d'atténuation et de vitesse) grâce à l'utilisation de transducteurs focalisés, les images ainsi obtenues étant comparables à celles obtenues par scanner.

### 3/ Estimation du coefficient d'atténuation et de la section efficace de rétrodiffusion en réflexion, en fonction de la fréquence.

Les différentes étapes de ce mode de traitement des signaux transmis sont les suivantes :
- Les transducteurs sont à la position X et Y ;
- Le transducteur T2 (ou T3, ou T1, ou T'1) émet
- Le transducteur T2 (ou T3, ou T1, ou T'1) assure la réception du signal échographique rétrodiffusé par l'architecture interne de l'os, ce signal étant ensuite amplifié, numérisé, et transféré sur ordinateur .
- On effectue une analyse spectrale glissante du signal échographique, et une estimation des spectres en fonction de la profondeur ;
- On calcule les centroïdes spectraux en fonction de la profondeur ;
- On calcule le coefficient d'atténuation en fonction de la fréquence (dB/cm.MHz) en réflexion ;
- On calcule la différence spectrale entre le spectre du signal et un spectre de référence, puis l'on calcule la section efficace de rétrodiffusion (en dB) en fonction de la fréquence en réflexion :
- On calcule le coefficient de rétrodiffusion (dB/MHz) en réflexion et
- On calcule le coefficient intégral de rétrodiffusion (dB.MHz) en réflexion. Le balayage du faisceau ultrasonore permet de réaliser une exploration de la totalité du volume osseux. Les coefficients d'atténuation et de rétrodiffusion sont calculés pour chaque position des transducteurs. On peut ensuite estimer une moyenne locale des paramètres à l'intérieur d'une région d'intérét sélectionnée par l'opérateur.

L'atténuation peut être estimée à partir du signal échographique radiofréquence échantilloné. Le principe du calcul de l'atténuation repose sur une analyse tempsfréquece du signal échographique.

L'invention permet ainsi de mesurer la section efficace de rétrodiffusion en fonction de la fréquence. On sait en effet que les propriétés physiques des tissus osseux sont mises en évidence par cette mesure.

Il résulte de la lecture de la description qui précède que l'invention apporte une solution aux difficultés posées par la mise en oeuvre des appareils ultrasonores d'analyse des os actuellement sur le marché :
- le dispositif de balayage automatique du faisceau ultrasonore permet de résoudre le problème du positionnement précis de l'os et du positionnement de la région de mesure où d'intérêt.
- L'utilisation de transducteurs focalises permet d'obtenir des images de bonne qualité ;
- Elle permet d'obtenir des mesures ultrasonores en transmission à travers l'os et en réflexion ;
- Elle permet de recueillir et de traiter des signaux réfléchis et/ou diffusés par l'architecture interne de l'os ce qui apporte une information complémentaire à celles se trouvant déjà dans les signaux qui ont été transmis à travers l'os ; Ainsi, le procédé et le dispositif objets de l'invention apportent une quantité d'informations bien supérieure à celle fournie par les dispositifs selon l'état antérieur de la technique, tout en offrant une précision une reproductibilité et une sensibilité meilleures.

Parmi les domaines d'application de l'invention, on a spécifié ci-dessus l'ostéoporose. Dans cette application, l'invention apporte une méthode physique non traumatique d'évaluation in vivo de la qualité osseuse (masse, rigidité, architecture). On peut ainsi apprécier de façon quantitative le risque de fracture associé à une diminution de la résistance des os, cette diminution étant comme on le sait, une conséquence des phénomènes de déminéralisation et de modification de l'architecture osseuse rencontrés dans l'ostéoporose.

La description faite ci-dessus d'exemples de réalisation de l'invention ne se limite pas aux mesures de calcaneum et de la rotule : il existe bien entendu d'autres domaines d'application de l'invention particuliérement en vue de la surveillance du degré de minéralisation du squelette, notamment afin de suivre l'évolution de l'architecture osseuse ou de l'élasticité de la structure osseuse. Parmi ces applications, on peut citer notamment :
- La maturation squelettique chez les nouveaux nés ;
- Les ostéoporoses secondaires ostéomalacie etc...
- La surveillance des chevaux de course .
- La caractérisation de pièces osseuses in vitro.

## Revendications

Il demeure bien entendu que la présente invention n'est pas limitée aux modes de mise en oeuvre ni aux exemples de réalisation décrits et/ou représentés ici, mais qu'elle en englobe toutes les variantes. Ainsi l'exemple de réalisation décrit ici se réfère à des mesures effectuées en immersion. Cependant, sans sortir du cadre de la présente invention, les mesures peuvent être effectuées par contact, en utilisant un réseau de transducteurs et un milieu de couplage, le balayage du faisceau ultrasonore pouvant être assuré électroniquement.

1. Procédé d'évaluation et de caractérisation in vivo des propriétés mécaniques ou architecturales des os, par propagation d'une onde ultra-sonore au-travers de l'os et l'étude de l'interaction de cette onde avec l'os, en vue de l'obtention d'images paramétriques selon lequel :
- on utilise au moins un transducteur focalisé émettant un faisceau ultra-sonore au travers de l'os ;
- on réalise un balayage dudit faisceau dans un plan perpendiculaire à la direction de propagation du faisceau ultrasonore ;
- on recueille les signaux transmis au-travers de l'os et/ou réfléchis par les faces de l'os et/ou diffusés par les structures internes de l'os ;
- on met en mémoire les signaux obtenus et,
- on traite les signaux ainsi stockés pour la mesure de la vitesse de propagation du faisceau ultra-sonore dans l'os, de l'épaisseur de l'os, du coefficient d'atténuation en transmission du faisceau ultrasonore et/ou des paramètres de réflexion pour l'estimation des coefficients de rétro-diffusion et d'atténuation.

2. Procédé selon la revendication 1 pour la réalisation, en mode transmission, d'images paramétriques d'atténuation en fonction de la fréquence et de la vitesse de propagation du faisceau ultrasonore, dans lequel on utilise une paire de transducteurs focalisés, placés en vis à vis et fonctionnant à basse fréquence, c'est-à-dire à une fréquence centrale comprise entre 100 kHz et 3 MHz, et de préférence entre 100 kHz et 1MHz.

3. Procédé selon la revendication 1 pour la réalisation en mode échographique d'images de réflectivité, dans lequel on utilise un transducteur focalisé ou une paire de transducteurs focalisés, placés en vis à vis, et fonctionnant avec une fréquence centrale comprise entre 100 kHz et 10 MHz.

4. Procédé selon la revendication 1 appliqué à la réalisation en mode échographique d'images de réflectivité et à l'estimation des coefficients d'atténuation et/ou de rétrodiffusion en fonction de la fréquence du faisceau ultrasonore, et également en vue de la réalisation, en transmission, d'images paramétriques d'atténuation en fonction de la fréquence et de la vitesse de propagation dans lequel on utilise une paire de transducteurs placés en vis-à-vis et fonctionnant à basse fréquence, c'est-à-dire à une fréquence centrale comprise entre 100 kHz et 1 MHz et, un transducteur focalisé ou une paire de transducteurs focalisés placés en vis à vis et fonctionnant à haute fréquence, c'est-à-dire à une fréquence centrale comprise entre 1 MHz et 10 MHz.

5. Procédé selon l'une quelconque des revendications 1 à 4 dans lequel les mesures sont effectuées par contact, à l'aide d'un milieu de couplage.

6. Procédé selon l'une quelconque des revendications précédentes dans lequel on utilise un réseau de transducteurs ultrasonores avec lesquels la focalisation et le balayage du faisceau ultrasonore peuvent être effectués électroniquement.

7. Dispositif pour la mise en oeuvre du procédé selon l'une quelconque des revendications précédentes comportant des moyens d'émission et de réception ultrasonores (12, 14) placés en vis-à-vis de part et d'autre de l'os à analyser, un module d'acquisition incluant les moyens d'émission d'ultrasons et de réception des signaux ultrasonores après leur interaction dans l'os, un système de mise en mémoire des signaux ainsi obtenus et, un module de traitement desdits signaux pour l'estimation des paramètres acoustiques connus pour leur relation avec les propriétés mécaniques ou viscoélastiques de l'os, lesdits moyens d'émission et de réception ultrasonores étant focalisés et le dispositif comprenant des moyens permettant de réaliser un balayage du faisceau ultrasonore ainsi émis.

8. Dispositif selon la revendication 7 dans lequel le module de traitement des signaux comporte un ordinateur, une mémoire de masse et un ensemble de logiciels de traitement de signaux conçus pour l'exploitation des données provenant du module d'acquisition, ce module de traitement comportant un ensemble de fonctions permettant notamment d'effectuer le calcul du coefficient d'atténuation en transmission, le calcul de l'épaisseur de l'os traversé à l'endroit de la mesure, le calcul de la vitesse de propagation, le calcul du coefficient de rétrodiffusion en réflexion et le calcul du coefficient d'atténuation en réflexion.

9. Dispositif selon l'une des revendications 7 ou 8 dans lequel les moyens d'émission et de réception comportent des transducteurs focalisés (12, 14), comprenant un seul élément, placés en vis-à-vis.

10. Dispositif selon l'une quelconque des revendications 7 à 9 comportant une enceinte (10) remplie d'un liquide, tel que de l'eau, afin de réaliser les mesures en immersion.

## Claims

1. Method for the in vivo evaluation and characterisation of the mechanical or architectural properties of bones, through the propagation of an ultrasonic wave through the bone and the study of the interaction of this wave with the bone, with a view to obtaining parametric images, according to which:
- at least one focused transducer emitting an ultrasonic beam through the bone is used;
- the said beam is swept in a plane perpendicular to the direction of propagation of the ultrasonic beam;
- the signals transmitted through the bone and/or reflected by the faces of the bone and/or diffused by the internal structures of the bone are collected;
- the signals obtained are stored in memory, and
- the signals thus stored are processed for the measurement of the speed of propagation of the ultrasonic beam in the bone, the thickness of the bone, the coefficient of attenuation of the ultrasonic beam in transmission and/or the reflection parameters for estimating the backscatter and attenuation coefficients.

2. Method according to Claim 1 for producing, in transmission mode, parametric attenuation images as a function of the frequency and speed of propagation of the ultrasonic beam, in which a pair of focused transducers are used, placed opposite each other and operating at low frequency, that is to say at a centre frequency of between 100 kHz and 3 Mhz, and preferably between 100 kHz and 1 MHz.

3. Method according to Claim 1 for producing, in echographic mode, reflectivity images, in which a focused transducer or a pair of focused transducers is used, placed opposite, and operating with a centre frequency of between 100 kHz and 10 Mhz.

4. Method according to Claim 1 applied to the production, in echographic mode, of reflectivity images and to the estimation of the attenuation and/or backscatter coefficients as a function of the frequency of the ultrasonic beam, and also with a view to producing, in transmission, parametric attenuation images as a function of the frequency and speed of propagation, in which use is made of a pair of transducers placed opposite each other and operating at low frequency, that is to say at a centre frequency of between 100 kHz and 1 MHz, and a focused transducer or a pair of focused transducers placed opposite each other and operating at high frequency, that is to say at a centre frequency of between 1 MHz and 10 Mhz.

5. Method according to any one of Claims 1 to 4, in which the measurements are made by contact, using a coupling medium.

6. Method according to any one of the preceding claims, in which use is made of an array of ultrasonic transducers with which the focusing and scanning of the ultrasonic beam can be effected electronically.

7. Device for implementing the method according to any one of the preceding claims, having ultrasonic emission and reception means (12, 14) placed opposite each other on each side of the bone to be analysed, an acquisition module including the ultrasound emission means and the means for receiving the ultrasonic signals after their interaction in the bone, a system for storing in memory the signals thus obtained, and a module for processing the said signals for estimating the acoustic parameters known for their relationship with the mechanical or viscoelastic properties of the bone, the said ultrasonic emission and reception means being focused and the device comprising means for effecting a scanning of the ultrasonic beam thus emitted.

8. Device according Claim 7, in which the signal processing module includes a computer, a mass memory and a set of signal processing software packages designed for using the data coming from the acquisition module, this processing module having a set of functions notably for calculating the attenuation coefficient in transmission, calculating the thickness of the bone passed through at the measuring point, calculating the speed of propagation, calculating the backscatter coefficient in reflection and calculating the attenuation coefficient in reflection.

9. Device according to one of Claims 7 or 8, in which the emission and reception means include focused transducers (12, 14) comprising a single element, placed opposite each other.

10. Device according to any one of Claims 7 to 9, having a chamber (10) filled with a liquid, such as water, in order to effect the measurements in immersion.

## Patentansprüche

1. Verfahren zur Bewertung und Charakterisierung mechanischer oder struktureller Eigenschaften von Knochen in vivo, indem eine Ultraschallwelle durch den Knochen geschickt und die Wechselwirkung dieser Welle mit dem Knochen untersucht wird, um Parameterbilder zu erhalten, **gemäß welchem**
- mindestens ein fokussierter Ultraschallkopf verwendet wird, der ein Ultraschallwellenbündel durch den Knochen sendet,
- das Ultraschallwellenbündel in einer Ebene, die senkrecht zu seiner Ausbreitungsrichtung steht, einen Scanvorgang ausfuhren gelassen wird,
- die Signale empfangen werden, welche durch den Knochen hindurchgehen und/oder von den Knochenflächen reflektiert und/oder von den inneren Knochenstrukturen gestreut werden,
- die erhaltenen Signale gespeichert werden und
- die so gespeicherten Signale für die Messung der Ausbreitungsgeschwindigkeit des Ultraschallwellenbündels im Knochen, der Knochendicke, des Schwächungskoeffizienten bei Transmission des Ultraschallwellenbündels und/oder der Reflexionsparameter für die Ermittlung des Rückstreu- und des Schwächungskoeffizienten verarbeitet werden.

2. Verfahren nach Anspruch 1, um im Transmissionsmodus Parameterbilder der Schwächung in Abhängigkeit von Frequenz und Ausbreitungsgeschwindigkeit des Ultraschallwellenbündels zu gewinnen, in welchem ein Paar fokussierter Ultraschallköpfe eingesetzt wird, die einander gegenüber angeordnet sind und bei Niedrigfrequenz, d.h. einer Mittenfrequenz von 100 kHz bis 3 MHz und vorzugsweise 100 kHz bis 1 MHz, arbeiten.

3. Verfahren nach Anspruch 1, um im Echographiemodus Bilder aus dem Reflexionsgrad zu gewinnen, in welchem ein fokussierter Ultraschallkopf oder ein Paar fokussierter Ultraschallköpfe, die einander gegenüber angeordnet sind und bei einer Mittenfrequenz von 100 kHz bis 10 MHz arbeiten, eingesetzt wird.

4. Verfahren nach Anspruch 1, das auf die Gewinnung von Bildern aus dem Reflexionsgrad und die Ermittlung des Schwächungs- und/oder des Rückstreukoeffizienten in Abhängigkeit von der Frequenz des Ultraschallwellenbündels im Echographiemodus gerichtet ist und auch, um in Transmission Parameterbilder der Schwächung in Abhängigkeit von Frequenz und Ausbreitungsgeschwindigkeit zu gewinnen, in welchem ein Paar Ultraschallköpfe, die einander gegenüber angeordnet sind und bei Niedrigfrequenz, d.h. einer Mittenfrequenz von 100 kHz bis 1 MHz, arbeiten, und ein fokussierter Ultraschallkopf oder ein Paar fokussierter Ultraschallköpfe, die einander gegenüber angeordnet sind und bei Hochfrequenz, d.h. einer Mittenfrequenz von 1 MHz bis 10 MHz, arbeiten, verwendet werden.

5. Verfahren nach einem der Ansprüche 1 bis 4, in welchem die Messungen mittels eines Kopplungsmediums durch Kontakt durchgeführt werden.

6. Verfahren nach einem der vorhergehenden Ansprüche, in welchem ein Netz von Ultraschallköpfen verwendet wird, bei denen die Fokussierung und das Scannen mit dem Ultraschallwellenbündel elektronisch durchgeführt werden können.

7. Vorrichtung zur Durchführung des Verfahrens nach einem der vorhergehenden Ansprüche, welche Mittel (12, 14) für das Aussenden und den Empfang von Ultraschallwellen, die einander gegenüber auf beiden Seiten des zu vermessenden Knochens angeordnet sind, ein Erfassungsmodul, einschließlich den Mitteln für das Aussenden von Ultraschallwellen und den Empfang der Ultraschallsignale nach ihrer Wechselwirkung im Knochen, ein System für die Speicherung der so erhaltenen Signale und ein Modul für die Verarbeitung dieser Signale zur Ermittlung der bekannten Schallparameter, um diese mit den mechanischen oder viskoelastischen Knocheneigenschaften zu korrelieren, umfaßt, wobei die Mittel für das Aussenden und den Empfang der Ultraschallwellen fokussiert sind und die Vorrichtung Mittel enthält, welche es ermöglichen, daß das so ausgesendete Ultraschallwellenbündel einen Scanvorgang ausführt.

8. Vorrichtung nach Anspruch 7, in welcher das Modul für die Signalverarbeitung einen Computer, einen Massenspeicher und eine Gruppe von Rechenprogrammen zur Signalverarbeitung, welche für die Auswertung der Daten entwickelt worden sind, die vom Erfassungsmodul kommen, umfaßt, wobei dieses Verarbeitungsmodul eine Gruppe von Funktionen enthält, welche insbesondere die Berechnung des Schwächungskoeffizienten bei Transmission, der Dicke des durchstrahlten Knochens an der Meßstelle, der Ausbreitungsgeschwindigkeit, des Rückstreukoeffizienten bei Reflexion und des Schwächungskoeffizienten bei Reflexion erlauben.

9. Vorrichtung nach Anspruch 7 oder 8, in welcher die Mittel für die Aussendung und den Empfang ein einziges Element enthaltende fokussierte Ultraschallköpfe (12, 14) umfassen, die einander gegenüber angeordnet sind.

10. Vorrichtung nach einem der Ansprüche 7 bis 9, umfassend einen Behälter (10), der mit einer Flüssigkeit wie Wasser gefüllt ist, um die Messungen im eingetauchten Zustand durchzuführen.
